# EUROPEAN PATENT APPLICATION

(11) **EP 4 779 557 A1**
(43) Date of publication of application: **22.07.2026**
(21) Application number: 25223014.9
(22) Date of filing: 12.12.2025
(51) Int. Cl.: G06T 5/70, G06T 5/60

(54) **IMAGE PROCESSING APPARATUS, RADIOGRAPHIC SYSTEM, IMAGE PROCESSING METHOD, AND STORAGE MEDIUM**

(30) Priority: 15.01.2025 JP 2025005360
(71) Applicant: Canon Kabushiki Kaisha, Tokyo, 146-8501 (JP)
(72) Inventor: NISHII, Yuichi, Tokyo, 146-8501 (JP)
(74) Representative: Canon Europe Limited

(57) **Abstract**

An image processing apparatus includes an acquisition unit configured to acquire a moving image obtained using a radiation detector, a first image processing unit configured to perform first image processing on the acquired moving image, the first image processing including noise reduction processing using a first machine learning model and processing for outputting the acquired moving image to a display system, and a second image processing unit configured to perform second image processing on the acquired moving image, the second image processing including noise reduction processing using a second machine learning model and processing for outputting the acquired moving image to a system different from the display system.

## Description

### Technical Field

The present disclosure relates to an image processing apparatus, a radiographic system, an image processing method, and a storage medium.

### BACKGROUND

In recent years, a radiographic system including a detecting unit for detecting radiation such as X-rays is widely used in an industrial field, a medical field, and other fields. In particular, in an X-ray moving image capturing field, digital radiographic systems that convert incident X-rays into visible light by using a scintillator (e.g., phosphor) to acquire a moving image by using a semiconductor sensor have widely been spread. The moving image is a set of a plurality of still images continuously collected, and hereinafter, each of the still images in the moving image is referred to as a frame. The moving image is also referred to as a fluoroscopic image.

In such a radiographic system, diagnosis performance (index indicating value of diagnosis images) is enhanced by applying various kinds of image processing on the image acquired by the semiconductor sensor. Examples of the image processing include noise reduction processing.

Japanese Patent Laid-Open No. 2013-48782 describes a rule-based technique for performing adequate noise reduction. More specifically, in the rule-based technique, a rule for accurately determining motion from a moving image is created in consideration of influence of noise, and adequate noise reduction is performed by performing weighted addition of a plurality of frames of the moving image in a time-series manner based on a determination result.

Nowadays, high-performance noise reduction processing using a machine learning-based technique such as deep learning has also been practically used. For example, "FastDVDnet: Towards Real-Time Deep Video Denoising Without Flow Estimation", M Tassano, et. al, IEEE/CVF Conference on Computer Vision and Pattern Recognition (CVPR), 2020, pp. 1354 to 1363 describes a technique in which frames before and after a frame, which is a noise reduction target, are input and a noise reduction processed image is obtained using a trained neural network.

In capturing of the moving image (fluoroscopic image), real-time display of the moving image and output of the moving image (e.g., output of moving image to picture archiving and communication systems (PACS)) are both required.

### SUMMARY

The machine learning-based noise reduction processing may achieve an excellent noise reduction effect as compared with the rule-based noise reduction processing. However, for example, the machine learning-based noise reduction processing may take more time than the rule-based noise reduction processing. Thus, both real-time display of the moving image and output of the moving image may not be performable. As a result, convenience for a photographer who captures a moving image (fluoroscopic image) may be impaired.

The present disclosure is directed to an image processing apparatus that can perform machine learning-based noise reduction processing, and can perform both real-time display of a moving image and output of the moving image.

The present disclosure in its first aspect provides an image processing apparatus as specified in claim 1. Optional features are specified in claims 2 to 12.

The present disclosure in its second aspect provides a radiographic system as specified in claim 13.

The present disclosure in its third aspect provides an image processing method as specified in claim 14.

The present disclosure in its fourth aspect provides a storage medium as specified in claim 15.

Features of the present disclosure will become apparent from the following description of embodiments with reference to the attached drawings. The following description of embodiments is described by way of example.

### BRIEF DESCRIPTION OF THE DRAWINGS

Fig. 1 illustrates an example of a schematic configuration of a radiographic system according to a first embodiment.
Fig. 2 illustrates an example of a schematic configuration of a control unit according to the first embodiment.
Figs. 3A to 3C illustrate an example of a schematic configuration and an operation example of a trained model according to the first embodiment.
Fig. 4 illustrates a first example of a flow of the control unit according to the first embodiment.
Fig. 5 illustrates a second example of the flow of the control unit according to the first embodiment.

### DESCRIPTION OF THE EMBODIMENTS

Embodiments of the present disclosure are described in detail below with reference to drawings.

Dimensions, materials, shapes, relative positions of components, and/or the like described in the following embodiments are optional, and can be changed according to a configuration of an apparatus to which the present disclosure is applied or various conditions. In the drawings, elements that are the same or functionally similar to each other will also be denoted by the same reference numerals.

In the following, a radiographic system using X-rays that are examples of radiation will be described. The radiation may be X-rays or other radiation. In the following embodiments, a term "radiation" may include, for example, electromagnetic radiation such as X-rays and γ-rays, and particle radiation such as α-rays, β-rays, particle rays, proton rays, heavy ion rays, and meson rays.

In the following, a machine learning model indicates a learning model based on a machine learning algorithm. Examples of a specific machine learning algorithm include a nearest neighbor method, a Naive Bayes method, a decision tree, and a support vector machine. A neural network or deep learning may also be applicable. Any usable algorithm among the above-described algorithms can be appropriately applied to the following embodiment. Training data indicates a data set to be used for training of the machine learning model, and includes pairs of input data to be input to the machine learning model and ground truth data (supervised data) that is ground truth of an output result of the machine learning model.

A trained model indicates a model obtained by previously performing training on a machine learning model based on an optional machine learning algorithm such as deep learning, by using appropriate training data. Although the trained model is obtained by previously performing training using appropriate training data, the trained model is not excluded from performing further training, and can perform additional training. The additional training can be performed after an apparatus is installed at a usage destination.

### (First Embodiment)

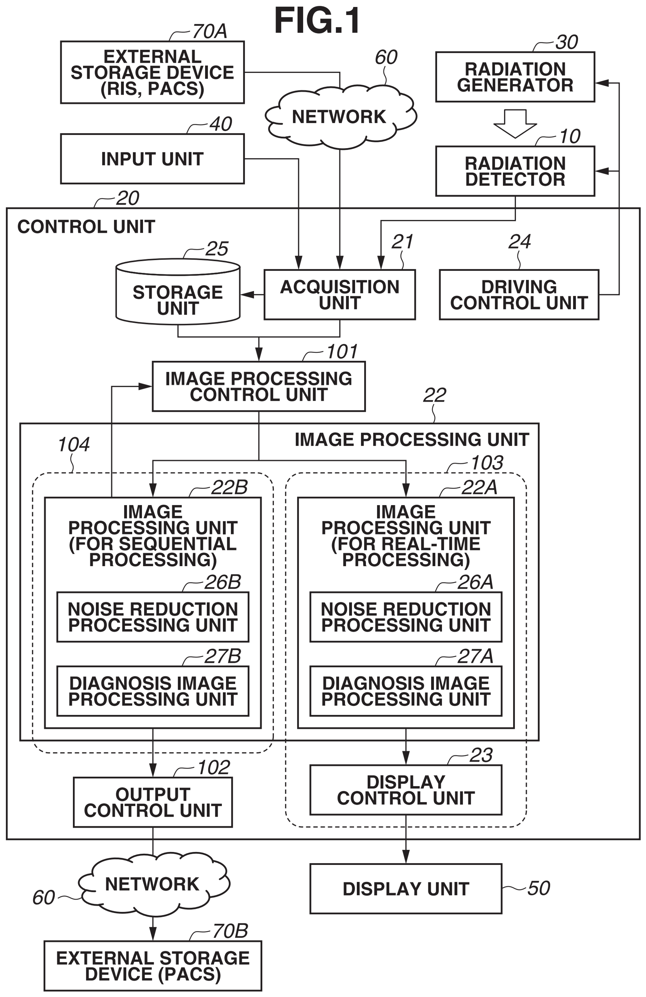

An example of a schematic configuration of a radiographic system according to a first embodiment of the present disclosure will now be described with reference to Fig. 1.

The radiographic system according to the first embodiment includes a radiation detector 10, a control unit 20, a radiation generator 30, an input unit 40, and a display unit 50.

The radiation generator 30 includes a radiation generation source such as an X-ray tube, and can emit radiation. The radiation detector 10 can detect radiation emitted from the radiation generator 30, and generate a radiation image corresponding to the detected radiation.

The control unit 20 is connected to the radiation detector 10, the radiation generator 30, the input unit 40, and the display unit 50. The control unit 20 can acquire the radiation image output from the radiation detector 10 and perform image processing on the radiation image, and control driving of the radiation detector 10 and the radiation generator 30. The control unit 20 can thereby control the radiation generator 30 to generate radiation of a predetermined imaging condition at an appropriate timing. The control unit 20 also enables still image capturing as well as moving image capturing at an optional frame rate. Examples of moving image capturing include fluoroscopy, continuous imaging, tomosynthesis imaging, digital subtraction angiography (DSA) imaging, and cone beam computed tomographic (CT) imaging.

The input unit 40 includes input devices such as a mouse, a keyboard, a trackball, and a touch panel, and can input an instruction to the control unit 20 by being operated by an operator.

The display unit 50 includes, for example, a monitor, and can display information and images output from the control unit 20, information input by the input unit 40, and/or the like. The display unit 50 may be a display system including a plurality of monitors. The display system includes, for example, a general-purpose monitor and a medical monitor.

The control unit 20 includes an acquisition means (e.g., acquisition unit 21), an image processing unit 22, a display control unit 23, a driving control unit 24, a storage means (e.g., storage unit 25), and an output control unit 102.

The acquisition unit 21 can acquire the radiation image output from the radiation detector 10, various kinds of information input by the input unit 40, and/or the like. The acquisition unit 21 can also acquire a radiation image, patient information, and/or the like from an external storage device 70A such as picture archiving and communication systems (PACS) and radiology information systems (RIS), via a network 60.

The image processing unit 22 includes noise reduction processing units 26 (26A and 26B) and diagnosis image processing units 27 (27A and 27B), and can perform image processing according to the present embodiment on the radiation image acquired by the acquisition unit 21. The image processing unit 22 can also perform reconstruction processing for creating a tomographic image, performed in tomosynthesis imaging or cone beam CT imaging.

The first embodiment describes an example in which a first image processing means (e.g., image processing unit 22A) for real-time processing and a second image processing means (e.g., image processing unit 22B) for sequential processing are provided as the image processing unit 22. The image processing unit 22A for real-time processing performs image processing every time a moving image is acquired. The image processing unit 22B for sequential processing does not perform image processing every time a moving image is acquired, but periodically performs image processing. The processing for periodically performing the image processing is also referred to as batch processing.

The image processing unit 22A includes the noise reduction processing unit 26A and the diagnosis image processing unit 27A. The image processing unit 22B includes the noise reduction processing unit 26B and the diagnosis image processing unit 27B. In the first embodiment, the noise reduction processing unit 26A and the noise reduction processing unit 26B perform noise reduction processing using the same machine learning model.

The noise reduction processing unit 26A and the noise reduction processing unit 26B may perform noise reduction processing using different machine learning models. For example, the machine learning model of the noise reduction processing unit 26A and the machine learning model of the noise reduction processing unit 26B may be different in parameters set to the machine learning model. Parameters relating to image processing (postprocessing) to be performed on an image output from the machine learning model may also be different therebetween.

Real-time image processing is an example of first image processing. The image processing unit 22A for real-time processing is an example of a first image processing unit. Sequential image processing is an example of second image processing. The image processing unit 22B for sequential processing is an example of a second image processing unit.

The reason why the image processing unit 22A for real-time processing and the image processing unit 22B for sequential processing are provided is to realize real-time display. In a case where the radiographic system receives a fluoroscopy start instruction, the radiographic system is required to rapidly display a fluoroscopic image received by the acquisition unit 21 on the display unit 50. In a case where one image processing unit 22 is provided, if the noise reduction processing using machine learning is performed for another purpose (reproduction, reconstruction, or output), it is difficult to rapidly display the fluoroscopic image on the display unit 50. The noise reduction processing using the machine learning takes a processing time, and accordingly occupies the image processing unit. As a result, it is difficult to rapidly display the fluoroscopic image on the display unit 50. In the first embodiment, the example in which two image processing units 22 are provided is described for facilitating understanding, but the configuration is not limited thereto. For example, four image processing units 22 (two image processing units for real-time processing and two image processing units for sequential processing) may also be provided.

The image processing unit 22A and the image processing unit 22B both perform image processing on an original image. The original image is an image after processing for correcting characteristics of the radiation detector (offset correction, gain correction, and defect correction) is performed on the image acquired by the radiation detector 10.

As the processing occupying the image processing unit other than the above-described noise reduction processing, there is reconstruction processing for creating a tomographic image, performed in tomosynthesis imaging or cone beam CT imaging. Examples of algorithm for reconstruction include a shift-and-add method, a filtered back projection (FBP) method, and a successive approximation method. In particular, the successive approximation method is known for a large calculation amount, and it is particularly anticipated that the successive approximation method occupies the image processing unit for a long time.

The display control unit 23 can control display of the display unit 50. For example, the display control unit 23 can cause the display unit 50 to display radiation images before and after image processing by the image processing unit 22, patient information, and/or the like.

The driving control unit 24 can control driving of the radiation detector 10, the radiation generator 30, and/or the like. The control unit 20 can therefore control driving of the radiation detector 10 and the radiation generator 30 by using the driving control unit 24. In other words, the control unit 20 can control capturing of a radiation image.

The storage unit 25 can store the information, the radiation image, and/or the like acquired by the acquisition unit 21. The storage unit 25 can also store an operating system (OS), device drivers for peripheral devices, and programs for realizing various kinds of application software including a program for performing processing and/or the like described below.

The output control unit 102 can output the radiation image to an external storage device 70B such as a picture archiving and communication system (PACS) via the network 60.

The image processing unit 22A for real-time processing performs image processing on a moving image represented by fluoroscopy or continuous imaging acquired by the acquisition unit 21, with image processing parameters previously set. The image processing unit 22A for real-time processing performs processing for rapidly transferring the processed image to the display control unit 23. By performing such processing as pipeline processing, real-time streaming display of the moving image can be performed. The image processing includes processing for converting the image into an image of bits (e.g., 8 bits or 32 bits) of the display unit so as to display the image on the display unit, and processing (e.g., gamma correction) for adjusting color brightness of the display unit.

The image processing unit 22B for sequential processing can perform reproduction and reconstruction of the image stored in the storage unit 25. In addition, the image processing unit 22B for sequential processing can sequentially perform image processing in order to perform output to the external storage device 70B and/or the like. The image processing unit 22B can also perform image processing using image processing parameters adjusted by the input unit 40. The image processing unit 22B for sequential processing basically sequentially performs the image processing in an instructed order, but can perform image processing by interruption according to a priority or the like.

An image processing control unit 101 can switch the image processing unit to be used between the image processing unit 22A for real-time processing and the image processing unit 22B for sequential processing depending on a condition.

A unit 103 including the image processing unit 22A for real-time processing and the display control unit 23 described in the first embodiment can be realized by a graphics processor (not illustrated and hereinafter referred to as a graphics processor 1). The graphics processor is, for example, a graphical processing unit (GPU). In the present embodiment, it is necessary to perform display control in the GPU. Thus, the GPU (e.g., graphics board) having output of the display unit 50 is included.

The graphics processor 1 (unit 103) transfers an image between the image processing in order to process the image at high speed. The transfer indicates, for example, transfer of an image between the noise reduction processing unit 26A and the diagnosis image processing unit 27A. Alternatively, the transfer indicates transfer between the image processing unit 22A and the display control unit 23. The transfer can be performed in a memory (e.g., video memory) of the graphics processor 1 (unit 103). In a case where architectures to be performed are different, the image is transferred, for example, after the image is copied. The method of transferring the image after the image is copied takes a time but can safely transfer the image. In addition, the transfer can be performed after the image is made exclusive (after image is encrypted). The method of transferring the image after the image is made exclusive takes less time than the method of transferring the image after the image is copied. In a case of the GPU, examples of the architecture include CUDA^{®}, DirectX^{®}, Direct3D^{®}, OpenCL^{®}, and OpenGL^{®}.

A unit 104 including the image processing unit 22B for sequential processing described in the first embodiment can be realized by a graphics processor (not illustrated and hereinafter referred to as a graphics processor 1). The graphics processor is, for example, a GPU. Unlike the graphics processor 1, the graphics processor 2 is not required to have the output of the display unit 50. The graphics processor 1 may be a central processing unit (CPU), a micro processing unit (MPU), a field-programmable gate array (FPGA), or the like.

Performance of the graphics processor 1 (unit 103) described in the first embodiment is higher than the performance of the graphics processor 2 (unit 104). In other words, the performance of the graphics processor 2 (unit 104) may be lower than or equivalent to the performance of the graphics processor 1 (unit 103), which is required to have high performance because of real-time processing necessary. The performance may be regarded as a processing time in a case where the same processing is performed. Alternatively, the performance may be regarded as an index such as floating-point operations per second (FLOPS) indicating processing performance. Alternatively, the performance may also be regarded as the number of cores mounted in the graphics processor, a version of the graphics processor, or the like.

The price of the graphics processor tends to increase as the performance of the graphics processor is high. Using a graphics processor lower in performance than the graphics processor 1 (unit 103) as the graphics processor 2 (unit 104) makes it possible to suppress the price of the control unit 20. The graphics processor 1 (unit 103) is an example of a first GPU. The graphics processor 2 (unit 104) is an example of a second GPU.

Processing other than the processing performed by the graphics processor 1 (unit 103) and the processing performed by the graphics processor 2 (unit 104) in the control unit 20 can be processed by the CPU because a processing amount is relatively small. However, the processing is not performable only by the CPU, and may be performed by the GPU, the MPU, the FPGA, or the like.

### (Configuration of Noise Reduction Processing Unit)

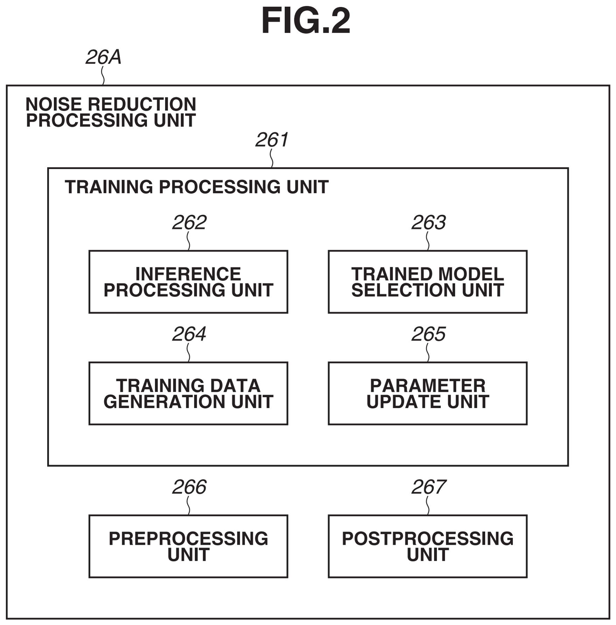

A configuration of the noise reduction processing unit 26A will be described with reference to Fig. 2. A configuration of the noise reduction processing unit 26B is also the same as the configuration of the noise reduction processing unit 26A.

As illustrated in Fig. 2, the noise reduction processing unit 26A includes a training processing unit 261. The training processing unit 261 includes an inference processing unit 262, and a trained model selection unit 263 as well as a training data generation unit 264, and a parameter update unit 265. The noise reduction processing unit 26A further includes a preprocessing unit 266 that converts the image input to the noise reduction processing unit 26A into an image in a format suitable for processing of the training processing unit 261, and a postprocessing unit 267 that applies appropriate processing to an output result of the training processing unit 261. With this configuration, the noise reduction processing unit 26A can perform training of a machine learning model for performing the noise reduction processing. The noise reduction processing unit 26A can also apply appropriate noise reduction processing to the radiation image by using the trained machine learning model. The noise reduction processing unit 26A may perform the noise reduction processing using trained parameters trained by another training apparatus. In other words, the noise reduction processing unit 26A may not have a configuration in which training of the machine learning model and the noise reduction processing (inference processing using trained parameters) are both performable.

The diagnosis image processing unit 27 can perform diagnosis image processing for converting the image subjected to noise reduction by the noise reduction processing unit 26A, into an image suitable for diagnosis. Examples of the diagnosis image processing include gradation processing for adjusting gradation of the image, enhancement processing for enhancing specific pixels in the image, and grid stripe reduction processing for reducing grid stripes in the image. The diagnosis image processing unit 27 may perform the gradation processing, the enhancement processing, the grid stripe reduction processing, and other processing depending on, for example, a region of interest (ROI) set in the radiation image. For example, the gradation processing may be performed to widen gradation of the region of interest, and the enhancement processing may be performed to enhance the region of interest. The region of interest may be set in response to an instruction of the operator, or may be set based on an imaging site, disease name information, observation information, or the like.

A configuration of the training processing unit 261 will now be described. The training processing unit 261 performs training processing to be applied when the machine learning model performs training. The training processing unit 261 includes the inference processing unit 262, the trained model selection unit 263, the training data generation unit 264, and the parameter update unit 265.

When the training processing is performed, the image subjected to the appropriate processing by the preprocessing unit 266 is input to the training processing unit 261, and the training data generation unit 264 generates training data. Here, a configuration example will be described in which an image added with artificial noise (input data) and an image not added with artificial noise (ground truth data) are used as a set of training data for training the noise reduction processing. The training data generation unit 264 performs processing for generating a set of training data by adding artificial noise that has been created by simulating characteristics of the radiation image to the input image. The noise added by the training data generation unit 264 may be noise reflecting a noise amount that has been calculated by the training data generation unit 264 and may be varied depending on manufacturing variation of the radiation detector 10.

The parameter update unit 265 performs processing for updating parameters of the machine learning model held by the inference processing unit 262 based on a calculation result of the inference processing unit 262 to the input data, and the ground truth data.

When the radiation image is input to the trained model that has performed training using the above-described training data, the inference processing unit 262 generates, by using inference processing, an image in which the image processing has been applied to the radiation image. The trained model selection unit 263 also selects the trained model used by the inference processing unit 262. At this time, the number of trained models obtained by the series of training processing of the training processing unit 261 may be plural, for example, for each model of the radiation detector 10. Alternatively, the number of trained models may be plural for each type of scintillator (e.g., phosphor) (not illustrated). Alternatively, the number of trained models may be plural for each type of an imaging sensor (not illustrated). Alternatively, the number of trained models may be plural for each of binning, sensitivity, a size of captured image, a frame rate, and an imaging procedure for a single model of the radiation detector 10. Among the plurality of trained models, at least one trained model used by the inference processing unit 262 is selected by the trained model selection unit 263.

A part of the training processing unit 261 is necessarily not included in the control unit 20. For example, the components other than the inference processing unit 262 and the trained model selection unit 263 may be provided on hardware (e.g., server) different from the control unit 20. The hardware creates the trained model by previously performing training by using appropriate training data. In this case, the control unit 20 may acquire the trained model by accessing the different hardware via the inference processing unit 262, and perform only processing using the trained model. Alternatively, the trained model may be previously provided in the noise reduction processing unit 26A, and the control unit 20 may perform only processing using the trained model.

Alternatively, the training processing unit 261 may be included in the control unit 20 to perform additional training using training data acquired after the radiographic system is installed in (sold to) a destination customer.

### (Configuration of Machine Learning Model)

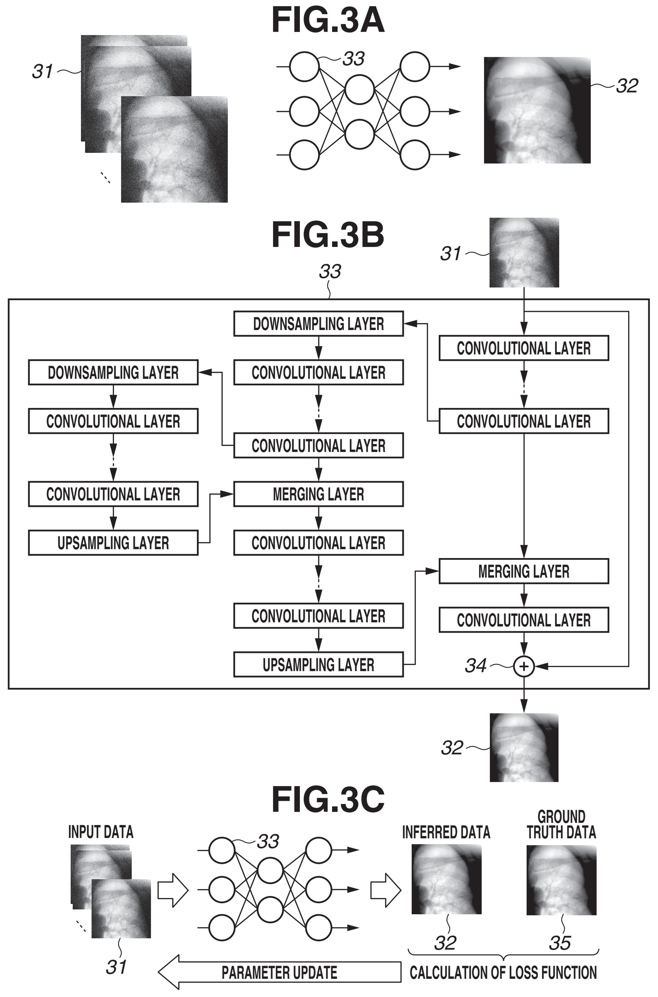

Next, an example of the machine learning model configuring the trained model according to the present embodiment will be described with reference to Fig. 3A to Fig. 3C. An example of the machine learning model used by the inference processing unit 262 according to the present embodiment is a multilayer neural network.

Fig. 3A illustrates a schematic configuration example of a neural network model according to the present embodiment. A configuration 33 of the neural network model illustrated in Fig. 3A is designed to output inference data 32 noise of which having been reduced depending on previously trained tendency, with respect to input data 31. The output inference data 32 noise of which having been reduced is based on training contents in the machine learning process. The neural network according to the present embodiment trains a features for classifying signals and noise included in the input radiation image. In the example illustrated in Fig. 3A, the input data 31 includes a current frame and one or more past frames. Alternatively, the input data 31 includes the current frame and one or more future frames. Alternatively, the input data 31 includes a frame group of the current frame, and any frames of one or more past frames and one or more future frames. The inference data 32 noise of which having been reduced is a frame obtained by reducing noise of the current frame. Note that a trained model may be configured in which the input data 31 includes only the current frame (one frame) and one frame is input.

For example, a convolutional neural network (CNN) can be used for at least a part of the multilayer neural network. Alternatively, a technique relating to an autoencoder or a technique relating to a vision transformer (ViT) may be used for at least a part of the multilayer neural network.

Here, an example will be described in which CNN is used as the machine learning model for noise reduction processing of the radiation image. Fig. 3B illustrates an example of the schematic configuration 33 of CNN configuring the neural network model according to the present embodiment. In the example of the trained model according to the present embodiment, when the input data 31 that is the radiation image is input, the inference data 32 can be output as the radiation image reduced in noise.

The CNN illustrated in Fig. 3B includes a plurality of layer groups performing processing for processing and outputting an input value group. Types of the layers included in the configuration 33 of the CNN include a convolutional layer, a downsampling layer, an upsampling layer, and a merging layer. The configuration 33 of the CNN can further include an addition layer 34, and configure a shortcut in which the input data is added before output. This enables the CNN to employ a configuration for training a difference between the input data and the output data, and to suitably handle a system targeting noise.

The convolutional layer is a layer performing convolutional processing on the input value group based on parameters such as a kernel size of a set filter, the number of filters, a stride value, and a dilation value. The number of dimensions of the kernel size of the filter may be changed depending on the number of dimensions of the input image.

The downsampling layer is a layer performing processing for making the number of an output value group less than the number of an input value group by thinning or merging the input values. More specifically, examples of such processing include max pooling processing.

The upsampling layer is a layer for performing processing for making the number of output values greater than the number of input values by duplicating the input values or adding values interpolated from the input values.

More specifically, examples of such processing include upsampling processing by deconvolution.

The merging layer is a layer for performing processing of inputting value groups, such as an output value group of a certain layer and a pixel value group configuring an image, from a plurality of sources to merge, couple or add the value groups.

When setting of parameters for layers and nodes configuring the neural network is different, reproducibility of tendency trained from the training data may be different in inference. Thus, caution is required. In other words, in many cases, since appropriate parameters are different depending on a mode at the time of implementation, the parameters can be changed as necessary.

Further, the CNN may obtain better characteristics by changing the parameters as described above, as well as by changing the configuration 33 of the CNN. The better characteristics are, for example, output of a radiation image noise of which has been reduced with higher accuracy, a short processing time, and a short time for training of a machine learning model.

The configuration 33 of the CNN used in the present embodiment is a U-net type machine learning model that includes a function of an encoder including a plurality of hierarchies including a plurality of downsampling layers, and a function of a decoder including a plurality of hierarchies including a plurality of upsampling layers. In the U-net type machine learning model, for example, skip connection can be used. In other words, positional information (space information) that has been obscured in the plurality of hierarchies configured as the encoder can be used in a hierarchy of the same dimension (hierarchy corresponding to dimension of encoder) in the plurality of hierarchies configured as the decoder.

Although not illustrated, as a modification of the configuration of the CNN, for example, layers of an activation function (e.g., rectifier linear unit (ReLu)) may be incorporated before and after the convolutional layer.

Characteristics of noise can be extracted from the input radiation image through these steps of the CNN.

The training processing unit 261 includes the parameter update unit 265. As illustrated in Fig. 3C, the parameter update unit 265 calculates a loss function from the inference data 32 that is obtained by applying the neural network model of the inference processing unit 262 to the input data 31 of the training data, and ground truth data 35 of the training data. Thereafter, the parameter update unit 265 performs processing for updating the parameters of the neural network model based on the calculated loss function.

The loss function indicates an error between the inference data 32 and the ground truth data 35.

The parameter update unit 265 can update filter coefficients and/or the like of the convolutional layer by using, for example, an error back-propagation method so as to reduce the error between the inference data 32 and the ground truth data 35 represented by the loss function. The error back-propagation method is a method of adjusting parameters and/or the like between nodes of the neural network so as to reduce the above-described error. Note that a method (dropout) of randomly deactivating units (neurons or nodes) configuring the CNN may be used for training.

The trained model used by the inference processing unit 262 may be a trained model generated using transfer learning. In this case, the trained model used for the noise reduction processing may be generated by performing, for example, the transfer learning on the machine learning model trained with radiation images of objects "O" to be inspected different in type or the like. By performing such transfer learning, it is possible to efficiently generate a trained model for the objects "O" to be inspected for which it is difficult to obtain a large amount of training data. The objects "O" to be inspected different in type or the like may be, for example, animals, plants, or objects of non-destructive inspection.

The GPU can perform efficient calculation by performing a large amount of data in parallel. In a case where training is performed a plurality of times by using the machine learning model using the above-described CNN, processing by the GPU is effective. Thus, the training processing unit 261 according to the present embodiment uses the GPU in addition to the CPU. More specifically, in the case of performing a training program including the machine learning model, the training is performed by the CPU and the GPU cooperating to perform calculation. In the training processing, calculation may be performed by only the CPU or the GPU. Each piece of the processing of the inference processing unit 262 may also be realized using the GPU as with the training processing unit 261.

The configuration of the machine learning model has been described above; however, the machine learning model is not limited to the above-described model using the CNN. Learning of the machine learning model used in the present embodiment may be learning belonging to machine learning using a model that can extract (represent), by itself, a feature amount of training data, such as an image, by training.

The training processing unit 261 according to the present embodiment can use an optional set of training data for training the noise reduction processing. The training processing unit 261 can use, for example, training data in which an image added with artificial noise is used as input data and an image not added with artificial noise is used as ground truth data. In addition, for example, training may be performed using training data in which an image before addition averaging processing is used as input data and an image after addition averaging processing is used as ground truth data, or training data in which an image before statistical processing such as maximum a posteriori probability (MAP) estimation processing is used as input data and an image after statistical processing is used as ground truth data. The example of supervised learning has been described above; however, the learning method is not limited thereto, and optional unsupervised learning or semi-supervised learning may be used.

### (First Flow of Control Unit in First Embodiment)

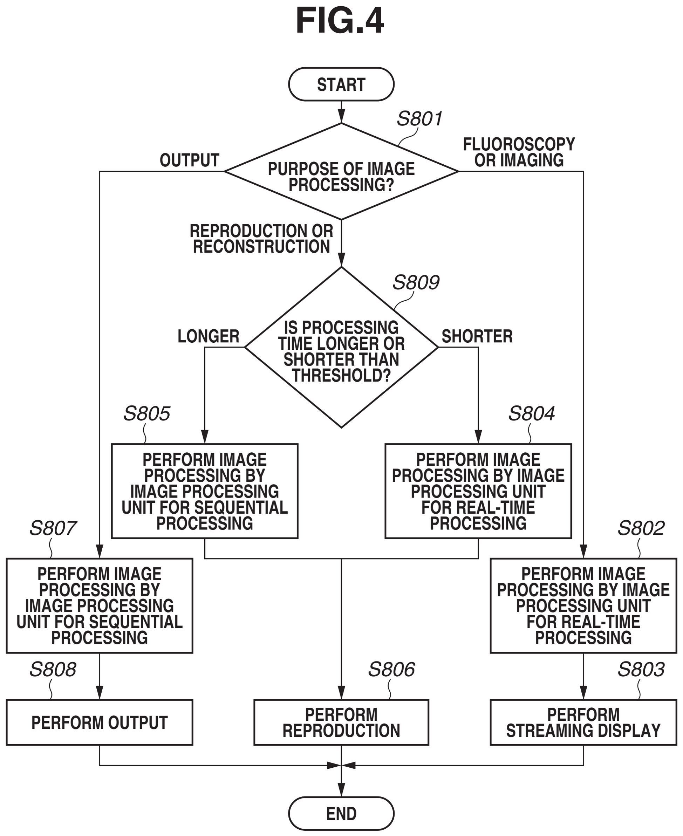

A first flow example of the control unit 20 of the radiographic system according to the first embodiment of the present disclosure will now be described below with reference to Fig. 4.

The image processing control unit 101 switches the image processing unit 22 to be used between the image processing unit 22A for real-time processing and the image processing unit 22B for sequential processing based on the condition as described above. In this flow, an example is described in which the image processing unit 22 is switched based on purpose of performing image processing.

In step S801, the image processing control unit 101 determines the purpose of image processing. The purpose of the image processing is defined (set), for example, when the radiographic system is constructed. Alternatively, for example, the purpose of the image processing is input by a photographer through a graphical user interface (GUI) provided on the display unit 50.

In a case where the purpose is image processing for fluoroscopy or imaging (FLUOROSCOPY OR IMAGING in step S801), the processing proceeds to step S802. In step S802, image processing is performed by the image processing unit 22A for real-time processing.

After the image processing is performed, the display unit 50 realizes, in step S803, a real-time streaming display under the control of the display control unit 23.

In a case where the purpose of the image processing is image processing for performing output to the PACS or the like (OUTPUT in step S801), the processing proceeds to step S807. In step S807, the image processing unit 22B performs the image processing for sequential processing.

After the image processing is performed, output to the PACS or the like is performed, in step S808, under the control of the output control unit 102.

In a case where the purpose of the image processing is reproduction of the image stored in the storage unit 25 or the like, or reconstruction of the tomographic image (REPRODUCTION OR RECONSTRUCTION in step S801), the processing proceeds to step S809.

In step S809, it is determined whether a predicted value of a processing time is longer or shorter than a threshold. The predicted value of the processing time is a value predicted from, for example, performance of the GPU and the processing contents. In a case where the processing time is shorter than the threshold (SHORTER in step S809), the processing proceeds to step S804. In step S804, the image processing is performed by the image processing unit 22A for real-time processing.

After the image processing is performed, a moving image is reproduced and displayed, in step S806, on the display unit 50 under the control of the display control unit 23.

In a case where it is determined that the predicted value of the processing time is longer than the threshold (LONGER in step S809), the processing proceeds to step S805. In step S805, the image processing is performed by the image processing unit 22B for sequential processing. In other words, the image processing is not performed by the image processing unit 22A for real-time processing.

After the image processing is performed, a moving image is reproduced and displayed, in step S806, on the display unit 50 under the control of the display control unit 23.

By performing determination based on the predicted value of the processing time in step S809, the processing in step S804 and the processing in step S805 can be switched by reconstruction algorithm. For example, in a case where the purpose of the image processing is reconstruction of tomosynthesis or reconstruction of cone beam CT, the processing in step S804 (real-time image processing) and the processing in step S805 (sequential image processing) can be switched by the reconstruction algorithm. More specifically, for example, in the case of a shift-and-add method or a filtered back projection method, the predicted value of the processing time is short, and accordingly, the real-time image processing is performed. In the case of a successive approximation method, the predicted value of the processing time is long, and accordingly, the sequential image processing is performed. By performing the processing having the long predicted value of the processing time with the image processing unit 22B for sequential processing, it is possible to prevent the image processing unit 22A for real-time processing from being long occupied. As a result, the fluoroscopic image can be rapidly displayed on the display unit 50.

### (Second Flow of Control Unit in First Embodiment)

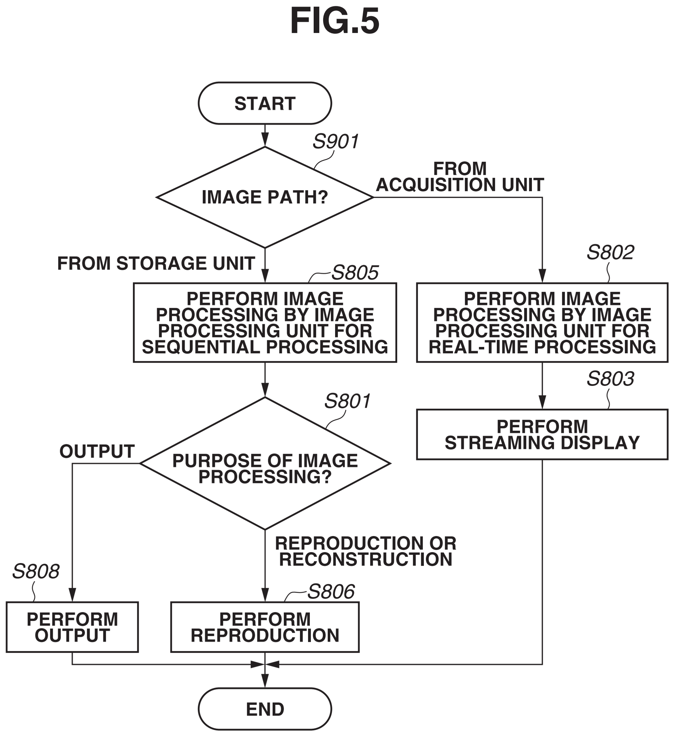

A second flow example of the control unit 20 of the radiographic system according to the first embodiment of the present disclosure will be described below with reference to Fig. 5.

The image processing control unit 101 switches the image processing unit 22 to be used between the image processing unit 22A for real-time processing and the image processing unit 22B for sequential processing depending on the condition as described above. In this flow, an example will be described in which the image processing unit 22 is switched depending on an image acquisition path (image acquisition source) as the condition.

In step S901, the image processing control unit 101 determines an image path. In a case where the image is an image acquired by the acquisition unit 21 (FROM ACQUISITION UNIT in step S901), the processing proceeds to step S802. In step S802, the image processing is performed by the image processing unit 22A for real-time processing because it is necessary to rapidly display the image on the display unit 50.

After the image processing is performed, the display unit 50 realizes, in step S803, real-time streaming display under the control of the display control unit 23.

In a case where the image is an image acquired from the storage unit 25 (FROM STORAGE UNIT in step S901), the processing proceeds to step S805. In step S805, the image processing is performed by the image processing unit 22B for sequential processing because it is unnecessary to rapidly display the image.

Thereafter, in step S801, purpose of the image processing is determined. Depending on a result of the determination, reproduction is performed in step S806 or output is performed in step S808. Operation in steps S801, S806, and S808 are similar to those described with reference to Fig. 4.

According to the present embodiment, it is possible to provide an image processing apparatus that can perform machine learning-based noise reduction processing, and can perform both real-time display of a moving image and output of the moving image.

### Other Embodiments

Embodiment(s) of the present disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and/or the like.

Various embodiments have been described in detail above but it will be understood that the present disclosure is not limited to these embodiments and encompasses all modifications, variants, alternatives and equivalents falling within the scope of the appended claims.

All of the features disclosed in this specification (including any accompanying claims, abstract and drawings), and/or all of the steps of any method or process so disclosed, may be combined in any combination, except combinations where at least some of such features and/or steps are mutually exclusive. Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

## Claims

1. An image processing apparatus, comprising:
acquisition means (21) configured to acquire a moving image obtained using a radiation detector (10);
first image processing means (22A) configured to perform first image processing on the acquired moving image, the first image processing including noise reduction processing using a first machine learning model and processing for outputting the acquired moving image to a display system; and
second image processing means (22B) configured to perform second image processing on the acquired moving image, the second image processing including noise reduction processing using a second machine learning model and processing for outputting the acquired moving image to a system different from the display system.

2. The image processing apparatus according to any preceding claim,
wherein the display system is a monitor, and
wherein the system different from the display system is picture archiving and communication systems, PACS.

3. The image processing apparatus according to any preceding claim, wherein the noise reduction processing included in the first image processing is real-time processing, and the noise reduction processing included in the second image processing is batch processing.

4. The image processing apparatus according to any preceding claim, wherein the first machine learning model used in the noise reduction processing included in the first image processing is the same as the second machine learning model used in the noise reduction processing included in the second image processing.

5. The image processing apparatus according to any preceding claim,
wherein the first image processing means (22A) includes a first graphics processing unit, GPU, and
wherein the second image processing means (22B) includes a second GPU lower in performance than the first GPU.

6. The image processing apparatus according to any preceding claim,
wherein, in a case where the acquired moving image is reproduced or reconstructed, and a predicted value of a processing time necessary for the reproduction or the reconstruction is shorter than a threshold, the first image processing means (22A) performs the reproduction or the reconstruction, and
wherein, in a case where the acquired moving image is reproduced or reconstructed, and the predicted value of the processing time necessary for the reproduction or the reconstruction is longer than the threshold, the second image processing (22B) means performs the reproduction or the reconstruction.

7. The image processing apparatus according to any preceding claim,
wherein, in a case where the acquired moving image is reproduced or reconstructed, and processing used for the reproduction or the reconstruction is a shift-and- add method or a filtered back projection method, the first image processing means (22A) performs the reproduction or the reconstruction, and
wherein, in a case where the acquired moving image is reproduced or reconstructed, and the processing used for the reproduction or the reconstruction is a successive approximation method, the second image processing means (22B) performs the reproduction or the reconstruction.

8. The image processing apparatus according to claim 6, wherein the acquired moving image is a moving image obtained by performing tomosynthesis imaging or computed tomographic, CT, imaging.

9. The image processing apparatus according to any preceding claim, further comprising storage means (25) for storing the moving image,
wherein the image processing apparatus performs the first image processing or the second image processing on the moving image acquired from the storage means (25) by using the second image processing means (22B).

10. The image processing apparatus according to any preceding claim, wherein the acquired moving image is a moving image subjected to at least one of offset correction, gain correction, or defect correction for correcting characteristics of the radiation detector.

11. The image processing apparatus according to any preceding claim, wherein the first image processing means performs image processing by using a preset image processing parameter, and the second image processing means performs image processing by using an image processing parameter set by operation of an operator.

12. The image processing apparatus according to any preceding claim, wherein
the processing for outputting the acquired moving image to the display system included in the first image processing comprises outputting an image subjected to noise reduction by the first machine learning model to the display system, and/or
the processing for outputting the acquired moving image to the system different from the display system included in the second image processing comprises outputting an image subjected to noise reduction by the second machine learning model to the system different from the display system.

13. A radiographic system, comprising:
the radiation detector (10); and
the image processing apparatus according to any of claims 1 to 12, communicably connected to the radiation detector (10).

14. An image processing method of an image processing apparatus, the image processing method comprising:
acquiring a moving image obtained using a radiation detector (10);
performing (S802) first image processing on the acquired moving image, the first image processing including noise reduction processing using a first machine learning model and processing for outputting the acquired moving image to a display system; and
performing (S807) second image processing on the acquired moving image, the second image processing including noise reduction processing using a second machine learning model and processing for outputting the acquired moving image to a system different from the display system.

15. A non-transitory computer-readable storage medium storing a program for causing a computer to perform the image processing method according to claim 14.
